# EUROPEAN PATENT SPECIFICATION

(11) **EP 2 903 507 B1**
(45) Date of publication and mention of the grant of the patent: **11.01.2017**
(21) Application number: 13843508.6
(22) Date of filing: 04.10.2013
(51) Int. Cl.: A61B 5/04, A61B 5/12, A61B 5/0492, H04R 25/00, A61N 1/36

(54) **ELECTROMECHANICAL MEASUREMENT OF STAPEDIUS MUSCLE/TENDON ACTIVITY**
ELEKTROMECHANISCHE MESSUNG DER AKTIVITÄT VON STAPEDIUSMUSKELN/-SEHNEN
MESURE ÉLECTROMÉCANIQUE DE L'ACTIVITÉ DES MUSCLES/TENDONS STAPÉDIENS

(30) Priority: 04.10.2012 DE 102012218153
(43) Date of publication of application: 12.08.2015
(73) Proprietor: MED-EL Elektromedizinische Geraete GmbH, Innsbruck 6020 (AT)
(72) Inventor: HESSLER, Roland, A-6020 Innsbruck (AT); DHANASINGH, Anandhan, A-6020 Innsbruck (AT); DEL CARMEN FUENTES, Maria, A-6020 Innsbruck (AT); PAU, Hans Wilhelm, 18055 Rostock (DE); OVARI, Attila, 18055 Rostock (DE); BEHREND, Detlef, 18119 Rostock-Warnemünde (DE); WARKENTIN, Mareike, 18059 Rostock (DE); SPECHT, Olaf, 18057 Rostock (DE); SCHMIDT, Wolfram, 18109 Rostock (DE)
(74) Representative: Lucke, Andreas
(86) International application number: PCT/US2013/063383
(87) International publication number: WO 2014/055823

(56) References cited:
- US-A- 6 157 861
- US-A1- 2011 137 180
- US-A1- 2011 307 042
- US-B1- 6 208 882
- US-B2- 6 485 408
- US-B2- 7 792 587
- US-B2- 8 121 687
- US-B2- 8 126 572

## Description

### TECHNICAL FIELD

The present invention relates to hearing prosthesis systems such as cochlear implant systems, and more specifically to measurement of stapedius tissue activity (i.e. contraction and/or stretching in the case of the stapedius muscle and movement in the case of the stapedius tendon) for such systems.

### BACKGROUND ART

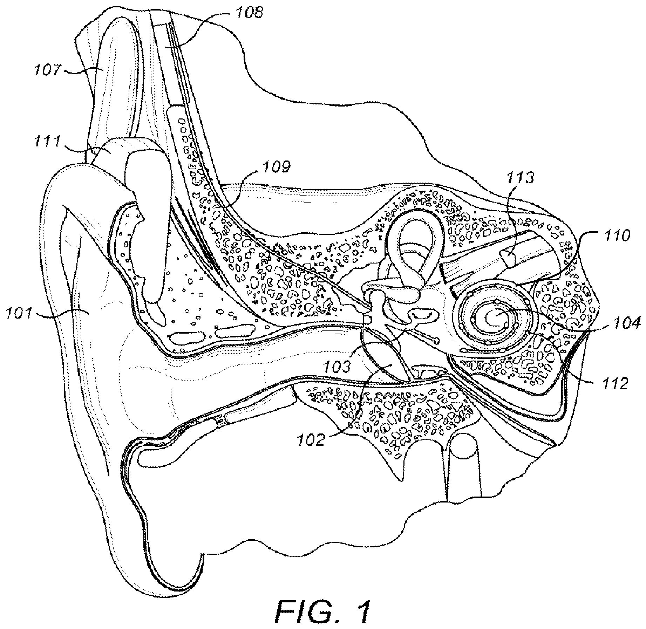

Most sounds are transmitted in a normal ear as shown in Figure 1 through the outer ear **101** to the tympanic membrane (eardrum) **102,** which moves the bones of the middle ear **103** (malleus, incus, and stapes) that vibrate the oval window and round window openings of the cochlea **104.** The cochlea **104** is a long narrow duct wound spirally about its axis for approximately two and a half turns. It includes an upper channel known as the scala vestibuli and a lower channel known as the scala tympani, which are connected by the cochlear duct. The cochlea **104** forms an upright spiraling cone with a center called the modiolus where the spiral ganglion cells of the acoustic nerve **113** reside. In response to received sounds transmitted by the middle ear **103,** the fluid-filled cochlea **104** functions as a transducer to generate electric pulses which are transmitted to the cochlear nerve **113,** and ultimately to the brain.

Hearing is impaired when there are problems in the ability to transduce external sounds into meaningful action potentials along the neural substrate of the cochlea **104.** To improve impaired hearing, auditory prostheses have been developed. For example, when the impairment is associated with the cochlea **104,** a cochlear implant with an implanted stimulation electrode can electrically stimulate auditory nerve tissue with small currents delivered by multiple electrode contacts distributed along the electrode.

Figure 1 also shows some components of a typical cochlear implant system which includes an external microphone that provides an audio signal input to an external signal processor **111** where various signal processing schemes can be implemented. The processed signal is then converted into a digital data format, such as a sequence of data frames, for transmission into the implant **108.** Besides receiving the processed audio information, the implant **108** also performs additional signal processing such as error correction, pulse formation, etc., and produces a stimulation pattern (based on the extracted audio information) that is sent through an electrode lead **109** to an implanted electrode array **110.** Typically, this electrode array **110** includes multiple electrodes on its surface that provide selective stimulation of the cochlea **104.**

Following surgical implantation, the cochlear implant (CI) must be custom fit to optimize its operation with the specific patient user. For the fitting process, it is important to know if an audible percept is elicited and how loud the percept is. Normally this information is gained using behavioral measures. For example, for each electrode contact the CI user is asked at what stimulation level the first audible percept is perceived (hearing threshold (THR)) and at what stimulation level the percept is too loud (maximum comfort level (MCL)). For CI users with limited auditory experiences or insufficient communication abilities (e.g., small children), these fitting parameters can be determined using objective measures.

One commonly used objective measure is the electrically evoked compound action potential (eCAP) which can be easily measured, but shows weak correlations with the MCL (r=0.57) and THR (r=0.55). *See, for example,* Miller et al., The Clinical Application Of Potentials Evoked From The Peripheral Auditory System, Hearing Research, 242(1-2), 184-197 (2008). The electrically evoked stapedius reflex threshold (eSRT) shows high correlations with the MCL. *See, for example,* Stephan, K. & Welzl-Müller, K., Post-Operative Stapedius Reflex Tests With Simultaneous Loudness Scaling In Patients Supplied With Cochlear Implants, Audiology, 39, 13-18 (2000) (r=0.92); and Polak, M.; Hodges, A. & Balkany, T ECAP, ESR and Subjective Levels For Two Different Nucleus 24 Electrode Arrays, Otology & Neurotology, 2005, 26, 639-645, (r= 0.93 - 0.95); But the eSRT electrical measurement is difficult to measure reliably; for example, movement artifacts of the impedance probe can introduce measurement artifacts. However, it has turned out that electromyographic (EMG) measurement of the activity of the stapedius musle is fairly difficult for various reasons.

US 2011/0137180 A1 discloses a system for fitting a cochlear prosthesis to an infant, comprising a sensor assembly (116) configured to measure a displacement of the stapedius that occurs in response to application of an electrical stimulation.

### SUMMARY

Embodiments of the present invention are directed to a device for measuring stapedius tissue activity (i.e., stapedius muscle and/or stapedius tendon activity) that uses a mechanoelectrical transducer having two ends. A static end is configured for attachment to the bony pyramid in the middle ear, and a dynamic end is configured for attachment to the stapedius tissue in the middle ear. The transducer generates a corresponding electrical sensing signal output when the stapedius tissue moves the dynamic end relative to the static end.

In specific embodiments, the transducer may include a mechanical strain gauge for generating the electrical sensing signal output., and there may be a substrate made of polyethylene terephthalate (PET) or polyaryletherketone (PAEK) for supporting the mechanical strain gauge. Or the transducer may use a piezoelectric foil for generating the electrical sensing signal output, and there may be a polyvinyl fluoride (PVF) foil substrate supporting the piezoelectric foil.

The transducer may form an elongated strip shape or a curved arch shape between the two ends. Or the transducer may form a curved loop shape with elongated parallel sections at the two ends that mechanically amplifies small movements of the stapedial tendon. In some embodiments at least one end may have a curved recess portion configured for attachment to the underlying anatomical structure. And some embodiments may include calibration means for isolating movement of the stapedius tissue from other movements in the middle ear of the patient.

Embodiments of the present invention also include a hearing implant fitting system and/or a hearing implant system (e.g., a cochlear implant, auditory brainstem implant, or middle ear implant) having a device according to any of the foregoing.

### BRIEF DESCRIPTION OF THE DRAWINGS

Fig. 1 shows anatomical structures of a human ear having a cochlear implant system.
Fig. 2 shows various structural features in the middle ear with a cochlear implant electrode passing through an opening in the round window membrane.
Fig. 3 shows a mechanoelectrical transducer attached to the stapedial tendon according to one specific embodiment of the present invention.
Fig. 4 A-D shows various structural geometries of alternative embodiments.
Fig. 5 shows the structural geometry of another embodiment of the present invention.

### DETAILED DESCRIPTION

Embodiments of the present invention are direct to a mechanoelectrical transducer device for measuring stapedius tissue activity (i.e., stapedius muscle activity and/or stapedius activity), specifically, contraction of the stapedial muscle in response to loud noise. A static end of the transducer is configured for attachment to the bony pyramid in the middle ear, and a dynamic end is configured for attachment to the stapedial tendon in the middle ear. Alternatively, the dynamic end may also be attached to the stapes directly, preferable to the stapes head. The transducer device generates a corresponding electrical sensing signal output when the stapedial tendon moves the dynamic end relative to the static end.

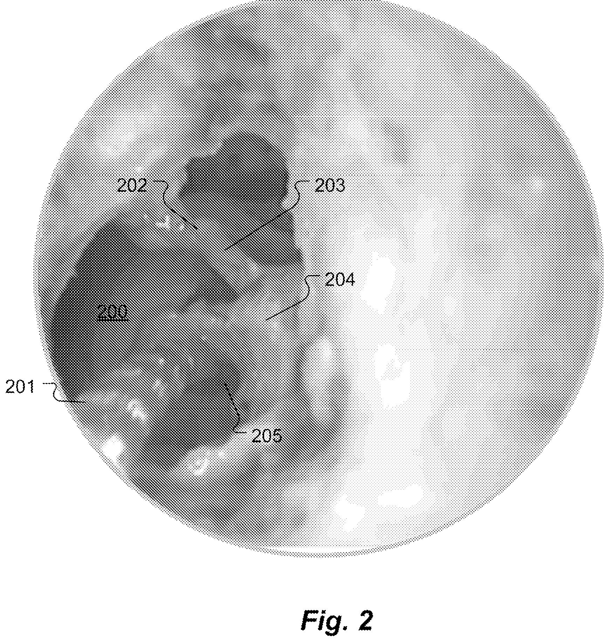

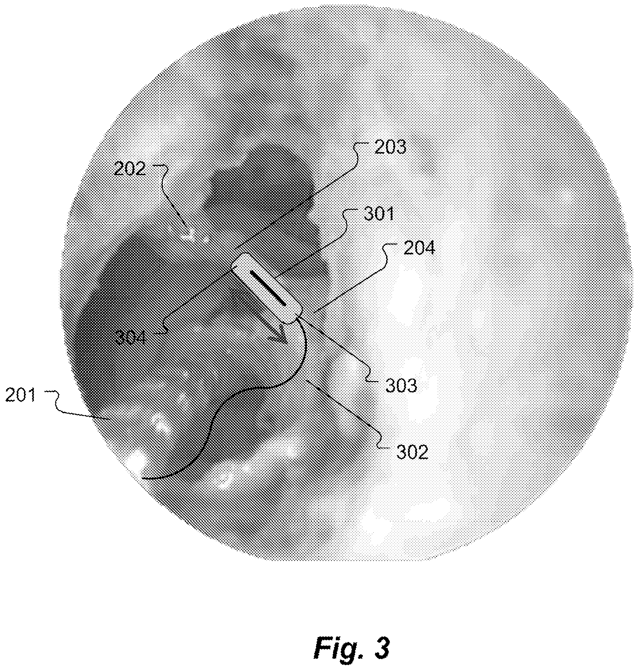

Figure 2 shows various structural features in the middle ear **200** of a patient with a cochlear implant electrode **201** that passes through an electrode opening **205** in the round window membrane on the outer surface of the patient's cochlea. The stapedial tendon **203** is connected at one end to the stapedial muscle inside the bony pyramid **204** (pyramidal eminence) and at the other end to the stapes head **202.** Figure 3 shows that anatomical context with the addition of a mechanoelectrical transducer **301** formed in an elongated strip shape that has a static end **303** configured for attachment to the bony pyramid **204,** and a dynamic end **304** configured for attachment to the stapedial tendon **203.** The electrical lead **302** from the transducer **301** joins the cochlear implant electrode **201** back away from the electrode opening **205** to isolate the transducer **301** from the effects of micro-movements of the electrode **201.** Alternatively rather than connecting the transducer **301** to the bony pyramid **204,** in some embodiments the static end **303** of the transducer **301** may be connected to the cochlear implant electrode **201.**

The transducer **301** generates a corresponding electrical sensing signal output when the stapedial tendon **203** moves the dynamic end **304** relative to the static end **303.** For example, the transducer **301** may specifically be a mechanical strain gauge that changes in electrical impedance when the stapedial muscle **203** contracts in response to loud noise (e.g., MCL threshold). In such embodiments, the transducer **301** may also include a strain gauge substrate, e.g., made of polyethylene terephthalate (PET) or polyaryletherketone (PAEK), for supporting the mechanical strain gauge.

Rather than a mechanical strain gauge, some embodiments may use a transducer **301** based on a piezoelectric foil that generates an electrical signal when the dynamic end **304** moves relative to the static end **303** when the stapedial muscle **203** stretches. In such embodiments, the transducer **301** have a foil substrate that supports the piezoelectric foil; for example, made of polyvinyl fluoride (PVF).

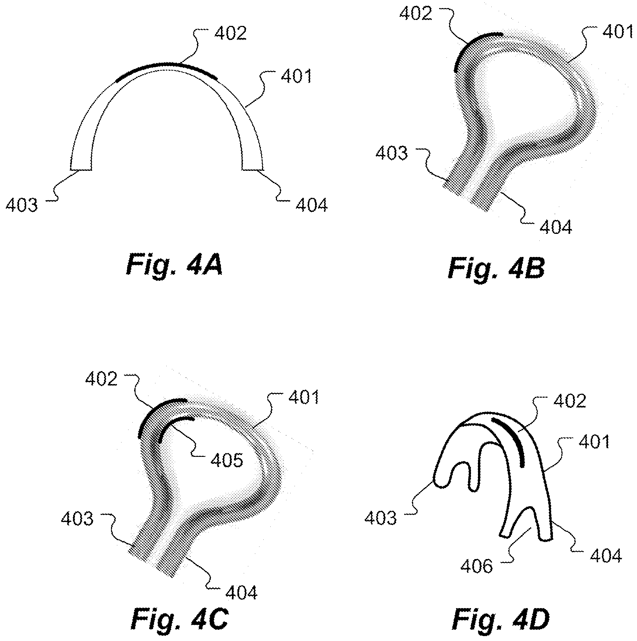

Embodiments of the present invention are not specifically limited to an elongated strip shape s shown in Fig. 3. For example, Figure 4A shows a curved arch shape transduce **401** having a mechanical strain gauge **402** at the top of the arch between the dynamic end **403** and the static end **404.** Figure 4B shows another embodiment of a transducer **401** having a curved loop shape with elongated parallel sections at the two ends **403** and **404.** The mechanical strain gauge **402** is located on the most highly curved part of the loop that is most sensitive to bending in response to small movements of the stapedial tendon **203;** in effect, acting as a mechanical amplifier. Figure 4C shows a similar embodiment with an additional strain gauge **405** on the opposite surface of the loop so that when the stapedial muscle **203** stretches in response to loud noise, one of the strain gauges **402/405** is compressed (producing increased impedance), while the other strain gauge **405/402** is stretched (producing decreased impedance).

As shown in Figure 4D, on some transducers **401** at least one end **403** and/or **404** may have a curved recess portion **406** configured for attachment to the underlying anatomical structure-the stapedial tendon **203** and/or the bony pyramid **204.** To affix an arch shaped transducer **401** such as the ones shown in Fig. 4A and 4D, the transducer **401** may use a shape memory material that is naturally biased to make the transducer **401** just slightly larger than the space where it is intended to be placed. The surgeon then compresses the ends of the transducer **401** towards each other to fit the transducer **401** in the desired location and position between the bony pyramid **204** and the stapedial tendon **203.** When the surgeon releases his grip, the ends expand back out to securely fix the transducer **401** in place.

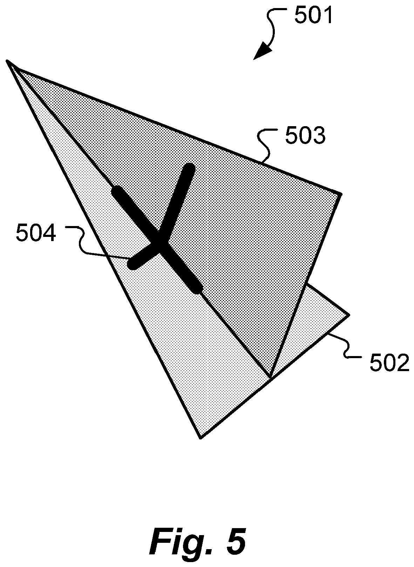

Figure 5 shows the structural geometry of another embodiment of an electromechanical transducer **501** for the measurement of stapedius muscle activity with two perpendicular surfaces **502** and **503** with a pair of corresponding perpendicular strain gauges **504** which provide calibration means for isolating movement of the stapedial tendon from other movements in the middle ear of the patient. Specifically, when the ear is exposed to any sound, the ossicular chain in the middle ear including the stapes head moves in response. When the incoming sound is very loud, the stapedius muscle contracts and the stapedial tendon pulls on the ossicular chain to dampen the signal. That stapedius reflex response to very loud sounds is the only movement of interest for a fitting related measurement. So an embodiment such as the one shown in Fig. 5 with multiple strain gauges that are perpendicular to each other can detect movements in different spatial dimensions, and the transducer **501** thereby can be calibrated to correlate the transducer output signal with contraction of the stapedial muscle and no other movements inside the middle ear.

Although various exemplary embodiments of the invention have been disclosed, it should be apparent to those skilled in the art that various changes and modifications can be made which will achieve some of the advantages of the invention without departing from the true scope of the invention as determined by the claims.

## Claims

1. A device for measuring middle ear stapedius tissue activity comprising:
a mechanoelectrical transducer having two ends:
i. a static end configured for attachment to the bony pyramid in the middle ear of a patient, and
ii. a dynamic end configured for attachment to stapedius tissue in the middle ear of the patient;
wherein the transducer generates a corresponding electrical sensing signal output when the stapedius tissue moves the dynamic end relative to the static end.

2. A device according to claim 1, wherein the transducer includes a mechanical strain gauge for generating the electrical sensing signal output.

3. A device according to claim 2, wherein the transducer includes a polyethylene terephthalate (PET) substrate supporting the mechanical strain gauge.

4. A device according to claim 2, wherein the transducer includes a polyaryletherketone (PAEK) substrate supporting the mechanical strain gauge.

5. A device according to claim 1, wherein the transducer includes a piezoelectric foil for generating the electrical sensing signal output.

6. A device according to claim 5, wherein the transducer includes a polyvinyl fluoride (PVF) foil substrate supporting the piezoelectric foil.

7. A device according to claim 1, wherein the transducer forms an elongated strip shape between the two ends.

8. A device according to claim 1, wherein the transducer forms a curved arch shape between the two ends.

9. A device according to claim 1, wherein the transducer forms a curved loop shape with elongated parallel sections at the two ends that mechanically amplifies small movements of the stapedius tissue.

10. A device according to claim 1, wherein at least one end has a curved recess portion configured for attachment of the at least one end.

11. A device according to claim 1, further including calibration means for isolating movement of the stapedial tissue from other movements in the middle ear of the patient.

12. A hearing implant fitting system having a device according to any of claims 1-11.

13. A hearing implant system having a device according to any of claims 1-11.

## Patentansprüche

1. Vorrichtung zum Erfassen von Aktivität des Stapediusgewebes im Mittelohr umfassend:
einen mechanoelektrischen Wandler, der zwei Enden aufweist:
i. ein statisches Ende, das zur Befestigung an der Knochenpyramide (bony pyramid) im Mittelohr eines Patienten geeignet ist, und
ii. ein dynamisches Ende, das zur Befestigung am Stapediusgewebe im Mittelohr eines Patienten geeignet ist;
wobei der Wandler als Ausgabe ein entsprechendes elektrisches Erfassungssignalerzeugt, wenn das Stapediusgewebe das dynamische Ende in Bezug auf das statische Ende bewegt.

2. Vorrichtung nach Anspruch 1, wobei der Wandler einen mechanischen Dehnmeßstreifen zur Erzeugung des elektrischen Erfassungssignals umfasst.

3. Vorrichtung nach Anspruch 2, wobei der Wandler ein Substrat aus Polyethylenterephthalat (PET) umfasst, das den mechanischen Dehnmeßstreifen hält.

4. Vorrichtung nach Anspruch 2, wobei der Wandler ein Substrat aus Polyaryletherketon (PAEK) umfasst, das den mechanischen Dehnmeßstreifen hält.

5. Vorrichtung nach Anspruch 1, wobei der Wandler eine piezoelektrische Folie zur Erzeugung des elektrischen Erfassungssignals umfasst.

6. Vorrichtung nach Anspruch 5, wobei der Wandler eine Folie aus Polyvinylfluorid (PVF) umfasst, die die piezoelektrische Folie hält.

7. Vorrichtung nach Anspruch 1, wobei der Wandler eine längliche Streifenform zwischen den zwei Enden bildet.

8. Vorrichtung nach Anspruch 1, wobei der Wandler eine gekrümmte Bogenform zwischen den zwei Enden bildet.

9. Vorrichtung nach Anspruch 1, wobei der Wandler eine gekrümmte Schleifenform mit länglichen parallelen Abschnitten an den zwei Enden bildet, die kleine Bewegungen des Stapediusgewebes mechanisch verstärkt.

10. Vorrichtung nach Anspruch 1, wobei mindestens ein Ende eine gekrümmte Aussparung aufweist, die zur Befestigung des mindestens einen Endes eingerichtet ist.

11. Vorrichtung nach Anspruch 1, die ferner ein Kalibrierungsmittel umfasst, um Bewegungen des Stapediusgewebes von anderen Bewegungen im Mittelohr des Patienten zu isolieren.

12. Gehörimplantatsbefestigungssystem umfassend einer Vorrichtung nach einem der Ansprüche 1 bis 11.

13. Gehörimplantatssystem umfassend einer Vorrichtung nach einem der Ansprüche 1 bis 11.

## Revendications

1. Dispositif pour mesurer l'activité du tissu stapédien de l'oreille moyenne comprenant :
un transducteur mécano-électrique ayant deux extrémités :
i. une extrémité statique configurée pour la fixation à la pyramide osseuse dans l'oreille moyenne d'un patient, et
ii. une extrémité dynamique configurée pour la fixation au tissu stapédien dans l'oreille moyenne du patient ;
dans lequel le transducteur génère une sortie de signal de détection électrique correspondante lorsque le tissu stapédien déplace l'extrémité dynamique par rapport à l'extrémité statique.

2. Dispositif selon la revendication 1, dans lequel le transducteur inclut un mesureur de tension mécanique pour générer la sortie de signal de détection électrique.

3. Dispositif selon la revendication 2, dans lequel le transducteur inclut un substrat en téréphtalate de polyéthylène (PET) supportant le mesureur de tension mécanique.

4. Dispositif selon la revendication 2, dans lequel le transducteur inclut un substrat en polyaryléthercétone (PAEK) supportant le mesureur de tension mécanique.

5. Dispositif selon la revendication 1, dans lequel le transducteur inclut une feuille piézoélectrique pour générer la sortie de signal de détection électrique.

6. Dispositif selon la revendication 5, dans lequel le transducteur inclut un substrat en feuille de poly(fluorure de vinyle) (PVF) supportant la feuille piézoélectrique.

7. Dispositif selon la revendication 1, dans lequel le transducteur a une forme de bande allongée entre les deux extrémités.

8. Dispositif selon la revendication 1, dans lequel le transducteur a une forme de voûte incurvée entre les deux extrémités.

9. Dispositif selon la revendication 1, dans lequel le transducteur a une forme de boucle incurvée à sections parallèles allongées aux deux extrémités qui amplifie mécaniquement de faibles mouvements du tissu stapédien.

10. Dispositif selon la revendication 1, dans lequel au moins une extrémité présente une partie de renfoncement incurvée configurée pour la fixation de l'au moins une extrémité.

11. Dispositif selon la revendication 1, incluant en outre des moyens d'étalonnage pour isoler le mouvement du tissu stapédien d'autres mouvements dans l'oreille moyenne du patient.

12. Système de mise en place d'implant auditif ayant un dispositif selon l'une quelconque des revendications 1 à 11.

13. Système d'implant auditif ayant un dispositif selon l'une quelconque des revendications 1 à 11.
